# EUROPEAN PATENT APPLICATION

(11) **EP 3 973 854 A1**
(43) Date of publication of application: **30.03.2022**
(21) Application number: 20290068.4
(22) Date of filing: 29.09.2020
(51) Int. Cl.: A61B 5/00, A61B 90/30, G01N 21/47, G01N 21/64, A61M 1/00, A61B 1/04

(54) **AN OPTICAL SPECTROSCOPY SYSTEM**

(71) Applicant: Koninklijke Philips N.V., 5656 AG Eindhoven (NL)
(72) Inventor: Hendriks, Bernardus Hendrikus Wilhelmus, 5656 AE Eindhoven (NL); Nachabé, Rami, 5656 AE Eindhoven (NL); Spliethoff, Jarich Willem, 5656 AE Eindhoven (NL); Babic, Drazenko, 5656 AE Eindhoven (NL)
(74) Representative: Philips Intellectual Property & Standards

(57) **Abstract**

The invention provides a system for investigating a tissue of a subject by way of diffuse reflectance spectroscopy, the tissue including a fluorescent agent adapted to absorb light in a first wavelength range and emit light in a second wavelength range, different to the first.

The system includes a cannula having a distal end to be positioned adjacent the tissue and an internal cavity for providing a suction force to the tissue. The system further includes a light source adapted to generate light at the first wavelength range and a fiber optic element to be positioned adjacent the tissue. The fiber optic element is adapted to receive a light signal from the fluorescent agent, the light signal comprising light in the first and second wavelength ranges.

The system further includes a processing system adapted to determine a concentration of the fluorescent agent in the tissue based on the light signal.

## Description

### FIELD OF THE INVENTION

The invention relates to the field of optical spectroscopy, and more specifically to the field of medical optical spectroscopy.

### BACKGROUND OF THE INVENTION

In a number of clinical applications the resection, or removal, of a given tissue may be aided using a contrast agent to help the clinician differentiate between the tissue to be removed and the surrounding tissue. This practice is widely used in the resection of tumors.

In the example of neurosurgical applications, the goal of surgical tissue resection for patients with malignant gliomas is the maximal safe resection of the tumor. However, a complete resection of the tumor is achieved in only a minority of patients. One reason for this limitation is the difficulty in distinguishing viable tumor tissue from healthy adjacent brain tissue during surgery at the tumor margin using conventional white-light microscopy.

Fluorescence-guided surgery (FGS) using 5-aminolevulinic acid (5-ALA) has been introduced in the treatment of malignant tumors to help the clinician distinguish between the tumor and the surrounding tissue, as discussed in C.G. Hadjipanayis, G. Widhalm and W. Stummer, "What is the surgical benefit of utilizing 5-ALA for fluorescence-guided surgery of malignant gliomas," Neurosurgery vol. 77 (215) pp. 663-673. Tissue fluorescence after oral administration of 5-ALA is associated with high sensitivity, specificity and positive predictive values for identifying malignant tumor tissue. 5-ALA is a natural metabolite in the human body that is produced with the hemoglobin metabolic pathway. Exogenous 5-ALA acts as a pro-agent that is orally administered and has unprecedented penetration of the blood brain barrier and tumor interface in brain tumors. Once 5-ALA is taken up by malignant glioma cells, it is metabolized into the fluorescent metabolite, protoporphyrin IX (PpIX). Elevated PpIX production within malignant brain tumor cells permits violet-red fluorescence visualization of malignant tumor tissue after excitation with 405nm wavelength blue light. The diagnostic accuracy of 5-ALA-induced tissue fluorescence in malignant gliomas is a key benefit for 5-ALA FGS.

Accordingly, FGS permits the intraoperative visualization of malignant tissue and provides the clinician with real-time guidance for differentiating tumor tissue from healthy brain tissue that is independent of neuronavigation and brain shift.

The problem with FGS methods as described above is that there is no way to measure the absolute fluorescent intensity of the tumor region in a robust way. Typically, when the intensity drops below a certain value the resection is stopped. Measuring the absolute fluorescence of the tumor region in a robust and controlled way is challenging due to: difficulties in providing constant illumination within a varying cavity in which the surface angle varies significantly; the specular reflection from the brain tissue wet-air interface; the angle of the camera that detects the fluorescent light; the reabsorption of the fluorescence light by the tissue itself; and the blood that may be present at the surface. These factors all make the absolute quantitative measurement of the 5-ALA concentration difficult.

A model based on diffusion theory first described in Farrell et al., "A diffusion theory model of spatially resolved, steady-state diffuse reflectance for the noninvasive determination of tissue optical properties in vivo," Med. Phys., vol. 19, no. 4, pp. 879-888, 1992 discusses determining the concentration of the fluorescent agent based on the absorption in the detection volume rather than measuring the emitted fluorescence. Knowing the absorption coefficient of the fluorescent agent as a function of the wavelength for the pure agent from the measured diffuse reflectance spectrum, the concentration of the fluorescent agent may be determined as described for instance in Rami et al., "Estimation of biological chromophores using diffuse optical spectroscopy: benefit of extending the UV-VIS wavelength range to include 1000 to 1600 nm," Biomed. Opt. Express, vol. 1, no. 5, pp. 1432-1442, 2010 and "Estimation of lipid and water concentrations in scattering media with diffuse optical spectroscopy from 900 to 1600nm," J. Biomed. Opt., vol. 15, no. 3, pp. 37015-10, 2010.

A problem with determining the concentration of the fluorescent agent based on absorption is that for some surgical applications, such as neurosurgery, constant force between the tissue and an imaging probe is difficult to achieve due to the weak and easy compressible nature off certain tissues, such as the brain. Furthermore, having a separate imaging probe for measuring the concentration of the agent in addition to a resection tool for removing the tissue of interest is not optimal as finding back the detected tumor tissue with the resection device is cumbersome for certain tissues due to the fact that some tissues are easily deformable.

There is therefore a need for a means of determining the fluorescent agent concentration in a tissue in a robust manner, where tool-tissue contact may be controlled without introducing additional tools during tissue resection such that the existing workflows are not interrupted.

### SUMMARY OF THE INVENTION

The invention is defined by the claims.

According to examples in accordance with an aspect of the invention, there is provided a system for investigating a tissue of a subject by way of diffuse reflectance spectroscopy, the tissue including a fluorescent agent adapted to absorb light in a first wavelength range and emit light in a second wavelength range, the second wavelength range being different to the first wavelength range, wherein the system comprises:
a cannula having a distal end to be positioned adjacent the tissue, wherein the cannula comprises an internal cavity configured to be connected to a pressure generator in order to generate a reduced pressure within the internal cavity, thereby providing a suction force to the tissue at the distal end of the cannula;
a light source adapted to generate light at the first wavelength range to be provided to the tissue;
a fiber optic element to be positioned adjacent the tissue and adapted to receive a light signal from the fluorescent agent, the light signal comprising light at the first wavelength range and light at the second wavelength range; and
a processing system, the processing system comprising an input for receiving the light signal, and wherein the processing system is adapted to determine a concentration of the fluorescent agent in the tissue based on a proportion of light at the first wavelength range in the light signal.

The system provides a means of accurately determining the concentration of a fluorescent agent within a tissue.

By providing suction, by way of the reduced pressure generated in the cannula, blood may be removed from the vicinity of the fiber optic element, thereby improving the accuracy of the determined concentration. Further, the suction may help to maintain good optical contact between the tissue and the fiber optic element by holding the tissue in place.

In an embodiment, determining the concentration of the fluorescent agent comprises measuring an absorbance of the fluorescent agent based on a comparison between the proportion of light generated by the light source in the first wavelength range and the proportion of light detected in the first wavelength range in the light signal.

In an embodiment, the fiber optic element is positioned relative to the cannula such that a distal end of the fiber optic element aligns with a distal end of the cannula.

In this way, good optical contact between the tissue and the fiber optic element may be maintained, thereby improving the accuracy of the determined concentration.

In a further embodiment, the fiber optic element is provided within the internal cavity of the cannula.

In this way, the positioning of the fiber optic element may be controlled in the same motion as the cannula, thereby simplifying the use of the fiber optic element. Further, by positioning the fiber optic element within the internal cavity, the suction force applied to the tissue by the cannula ensures that any blood in the vicinity of the fiber optic element is removed. In addition, the suction force generated by way of the cannula generates and maintains good optical contact between the tissue and the fiber optic element.

In an embodiment, the system further comprises a sleeve adapted to receive the cannula, wherein the fiber optic element is integrated into the sleeve.

In this way, the positioning of the fiber optic element may be controlled based on the motion of the cannula, thereby simplifying the use of the fiber optic element. By providing the fiber optic element in a sleeve separate from the cannula, the system may be implemented using existing suction cannulas, which are already in use in tissue investigation and resection procedures, by providing the sleeve containing the fiber optic element to the existing cannula system.

In an embodiment, the fiber optic element comprises a plurality of optical fibers.

In a further embodiment, the light source is coupled to a first optical fiber of the plurality of optical fibers, and wherein the input for receiving the light signal is coupled to a second optical fiber of the plurality of optical fibers.

In an embodiment, the light source comprises one or more LEDs, and wherein the input for receiving the light signal comprises a photodiode.

In an embodiment, the light source comprises a white light source, and wherein the system further comprises an optical filter adapted to filter out light outside of the first wavelength range.

In an embodiment, the processing system is further adapted to identify a boundary between the tissue and an adjacent tissue based on the determined concentration of the fluorescent agent in the tissue.

In this way, the extent of the tissue of interest may be identified automatically by the system.

In a further embodiment, the processing system is adapted to receive an initial reading of the adjacent tissue by way of the fiber optic element, and wherein determining the concentration of the fluorescent agent is further based on the initial reading.

In this way, the system may be calibrated based on the adjacent tissue, thereby increasing the accuracy of the identification of the boundary.

In an embodiment, the processing system is further adapted to generate an alert to be provided to a user based on the identified boundary.

In this way, the user may be informed of the presence of a tissue boundary.

In an embodiment, the system further comprises a display for displaying the alert to the user.

In an embodiment, the system further comprises a display for displaying the determined concentration of the fluorescent agent to a user.

In an embodiment, the system is adapted for use when the tissue is being resected.

In another aspect the present disclosure provides an arrangement comprising a fiber optic element (170) adapted to be connected to a system (100) for investigating a tissue (110) of a subject by way of diffuse reflectance spectroscopy, the tissue including a fluorescent agent adapted to absorb light in a first wavelength range and emit light in a second wavelength range, the second wavelength range being different to the first wavelength range, the fiber optic element comprising a connector for removably connecting the optical fiber element to a cannula such that it is positioned adjacent the tissue upon use of a cannula (120) having a distal end to be positioned adjacent tissue of a subject, wherein the cannula comprises an internal cavity (130) configured to be connected to a pressure generator in order to generate a reduced pressure within the internal cavity, thereby providing a suction force to the tissue at the distal end of the cannula and, optionally, wherein the connector comprises a sleeve for receiving the cannula and having attached thereto or integrated therein the optical fiber element. The sleeve, optical fiber element and/or the cannula as preferably defined as described herein, e.g. for example in relation to the system Preferably the arrangement also comprises the cannula.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a schematic representation of a system for investigating a tissue of a subject by way of diffuse reflectance spectroscopy,
Figure 2A to Figure 2C show examples of cannula for use in the system of Figure 1 according to aspects of the invention; and
Figure 3 shows a method for using the system of Figure 1.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for investigating a tissue of a subject by way of diffuse reflectance spectroscopy, the tissue including a fluorescent agent adapted to absorb light in a first wavelength range and emit light in a second wavelength range, different to the first.

The system includes a cannula having a distal end to be positioned adjacent the tissue and an internal cavity for providing a suction force to the tissue. The system further includes a light source adapted to generate light at the first wavelength range and a fiber optic element to be positioned adjacent the tissue. The fiber optic element is adapted to receive a light signal from the fluorescent agent, the light signal comprising light in the first and second wavelength ranges.

The system further includes a processing system adapted to determine a concentration of the fluorescent agent in the tissue based on the light signal.

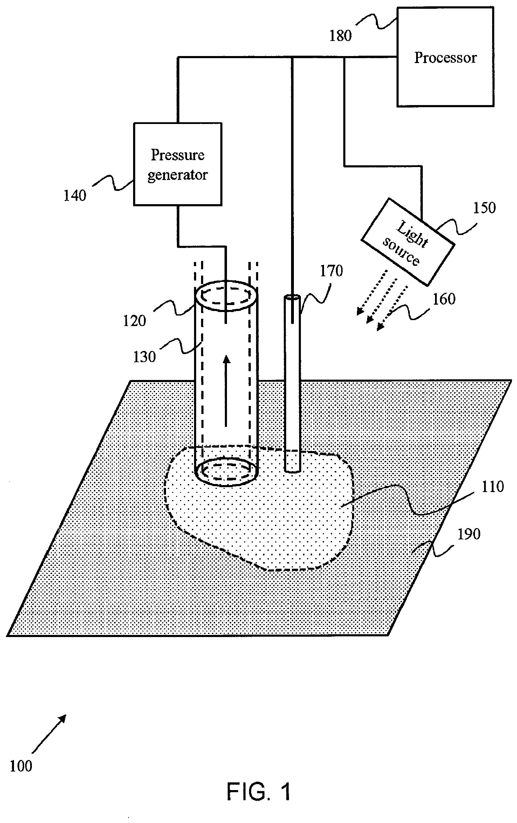

Figure 1 shows a system 100 for investigating a tissue 110 of a subject by way of diffuse reflectance spectroscopy. The tissue may be any tissue of a subject that includes a fluorescent agent adapted to absorb light in a first wavelength range and emit light in a second wavelength range, the second wavelength range being different to the first wavelength range. For example, the tissue may comprise: tumor tissue; brain tissue; lung tissue; organ tissue; muscle tissue; and the like.

The system 100 comprises a cannula 120 having a distal end to be positioned adjacent the tissue 110, wherein the cannula comprises an internal cavity 130 configured to be coupled to a pressure generator 140. The pressure generator is adapted to generate a reduced pressure within the internal cavity, thereby providing a suction force to the tissue at the distal end of the cannula. Put another way, the cannula and pressure generator form a suction cannula system for providing a suction force to the tissue of interest.

The system 100 further comprises a light source 150 adapted to generate light 160 in the first wavelength range to be provided to the tissue 110. As discussed above, the fluorescent agent present in the tissue will absorb light in the first wavelength range and emit light in a second, different, wavelength range. The light source may comprise any suitable source of light, such as: one or more LEDs, wherein the LEDS are adapted to generate light in the first wavelength range only; or a white light source, such as one or more white LEDs or a halogen lamp, which will generate light including at least the first wavelength range.

In the case where the light source is a white light source, the system may further comprise an optical filter adapted to filter out light outside of the first wavelength range. In other words, the optical filter may permit only light within the first wavelength range to pass through it. The optical filter may be positioned at any suitable location within the system along the optical path defined between the light source and the input for receiving the light signal.

Alternatively, the input for receiving the light signal at the processing system may comprise a detector adapted to resolve the intensity of the received light signal as a function of wavelength. The proportion of the signal within the first wavelength range may then be assessed to determine the concentration of the fluorescent agent in the tissue.

The system 100 also includes a fiber optic element 170 to be positioned adjacent the tissue. The fiber optic element is adapted to receive a light signal from the fluorescent agent within the tissue, the light signal comprising light at the first wavelength range and light at the second wavelength range. Put another way, the fiber optic element will receive incident light in both the first and second wavelength ranges, the light in the first wavelength range being diffuse reflected from the tissue being contacted by the fiber optic element and the light in the second wavelength range being emitted from the fluorescent agent within the tissue. The light source may be coupled to the fiber optical element for providing the light in the first wavelength range to the tissue.

The fiber optic element may be any type of waveguide, such as an optical fiber, for guiding the light signal from the tissue to the processing system.

The fiber optic element 170 is coupled to a processing system 180, the processing system comprising an input for receiving the light signal, such as a photodiode or a spectrometer. In the example of a spectrometer, the received light signal in the first wavelength range may be analyzed as a function of the wavelength. The processing system is adapted to determine a concentration of the fluorescent agent in the tissue based on a proportion of light at the first wavelength range in the light signal. The determined concentration may be indicative of whether the fluorescent agent is present or not and, apart from the presence of the fluorescent agent, the absolute concentration of the fluorescent agent in the tissue. Measuring the presence of the fluorescent agent means that the tissue is still present and further resection of the tissue is required. The proportion of light in the first wavelength range is indicative of the presence and concentration of the fluorescent agent in the tissue being investigated. The fluorescent agent concentration may be determined, for example, as described in Rami et al., "Estimation of biological chromophores using diffuse optical spectroscopy: benefit of extending the UV-VIS wavelength range to include 1000 to 1600 nm", 22 November 2010 / Vol. 18, No. 24 / OPTICS EXPRESS 1442.

Determining the concentration of the fluorescent agent may include measuring an absorbance of the fluorescent agent based on a comparison between the proportion of light generated by the light source in the first wavelength range and the proportion of light detected in the first wavelength range in the light signal. The output of the light source may be known, meaning that the proportion of light detected in the first wavelength range in the light signal obtained by way of the optical fiber may be compared to the known output in order to determine the absorbance of the fluorescent agent.

The concentration of the fluorescent agent within the tissue may be determined algorithmically using the proportion of light in the first wavelength range in the light signal. Further, the concentration of the fluorescent agent within the tissue may be determined using a machine learning algorithm.

A machine-learning algorithm is any self-training algorithm that processes input data in order to produce or predict output data. Here, the input data comprises a proportion of light in the first wavelength range in the light signal and the output data comprises a concentration of a fluorescent agent within a tissue.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example proportions of light in the first wavelength range. The training output data entries correspond to concentrations of the fluorescent agent in a tissue.

The system 100 may further comprise a display for displaying the determined concentration of the fluorescent agent to a user.

The system described above provides a suction cannula system equipped with an optical fiber connected to a processing system, such as an optical spectroscopy console (OSC), capable of determining the concentration of a fluorescent agent based, for example, on the absorbance of a fluorescent agent within the tissue being investigated measured by diffuse reflectance spectroscopy (DRS). The suction force of the cannula provides a means of establishing strong and consistent tissue contact between the optical fiber and the tissue, which improves the accuracy of the determined concentration. Further, the suction force provided by the cannula further mitigates the hindering light absorption properties of blood that would otherwise hamper of detection of the fluorescent agent. Most, if not all, of the blood that occurs within the region of the tissue may be removed from the region by the suction force of the cannula and, due to the suction-established optical fiber to tissue contact, there will be a reduction in the amount of blood pooling at the tip of the optical fiber.

The processing system 180 may be further adapted to identify a boundary between the tissue 110 and an adjacent tissue 190 based on the determined concentration of the fluorescent agent in the tissue.

By obtaining a concentration of the fluorescent agent, and in particular the change in the concentration during the resection procedure, the boundary of the tumor may be identified. The concentration of the fluorescent agent will drop to zero outside the tissue 110; however, this decrease in concentration will occur gradually over a transition zone. A significant drop in the concentration of the fluorescent agent may be indicative of the tissue boundary.

The processing system 180 may be adapted to generate an alert, such as a visual alert by way of a display or an audible alert by way of a speaker, to be provided to the user based on the identified boundary. For example, when a significant drop in the concentration of the fluorescent agent is identified, the user may be warned that the tissue boundary is close and additional care should be taken with the resection of the tissue, thereby sparing as much healthy brain tissue as possible.

The processing system 180 may be further adapted to receive an initial reading of the adjacent tissue 190 by way of the fiber optic element 170. In this case, determining the concentration of the fluorescent agent may be further based on the initial reading. Accordingly, the system may undergo a calibration using the initial reading obtained from the adjacent tissue as a baseline value against which the absorbance of the fluorescent agent may be evaluated, thereby improving the accuracy of the determination of the fluorescent agent concentration.

The fiber optic element may be positioned relative to the cannula such that a distal end of the fiber optic element aligns with a distal end of the cannula. The fiber optic element may be coupled to the cannula in order to maintain the alignment of the distal ends as the user moves the cannula. Thus, the fiber optic element may be integrated into a conventional suction cannula system without requiring the user to operate and position a separate piece of equipment.

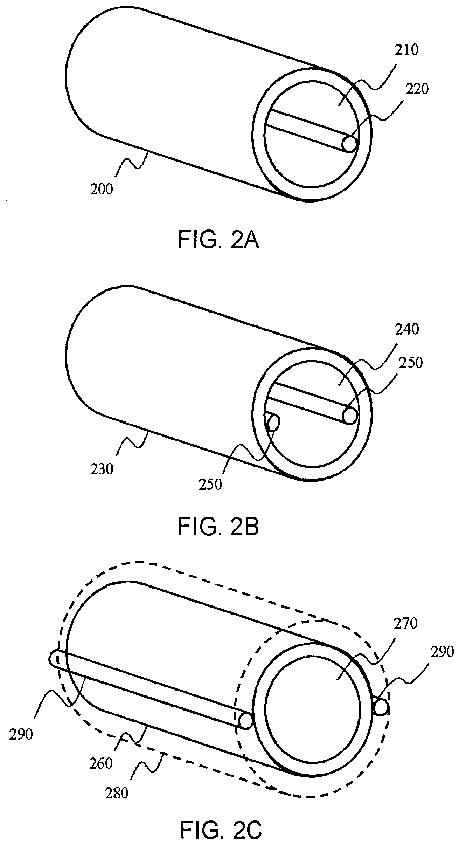

Figure 2A shows an example of a cannula 200 for use in a system such as the system described above with reference to Figure 1, wherein the cannula comprises an internal cavity 210 in which a fiber optic element 220 has been provided. As the fiber optic element 220 has been integrated along the internal cavity of cannula, the distal end of the fiber coincides with the distal end of the cannula. The fiber optic element may be integrated into the internal cavity of the cannula such that no additional tools, such as a separate fiber optic element requiring manipulation by the user, are introduced to the resection procedure. As the suction cannula is constantly used during the resection procedure, no additional tools will be required to perform the determination of the concentration of the fluorescent agent in the tissue, thereby enabling an uninterrupted workflow.

Figure 2B shows an example of a cannula 230 for use in a system such as the system described above with reference to Figure 1, wherein the cannula comprises an internal cavity 240 in which a plurality of optical fibers 250, which form part of the fiber optic element of the system, have been provided.

In the example shown in **Error! Reference source not found**.B, two optical fibers are integrated into the internal cavity of the cannula. The distal ends of the optical fibers are aligned with the distal end of the cannula. The cannula will provide a suction force to the tissue with a constant pressure, thereby holding the tissue against the optical fiber ends with a constant force, thereby creating a strong optical connection between the tissue and optical fibers, which is necessary for a robust readout of the diffuse reflectance spectrum of the tissue.

Figure 2C shows an example of a cannula 260 for use in a system such as the system described above with reference to Figure 1, wherein the cannula comprises an internal cavity 270. In addition, there is provided a sleeve 280 adapted to receive the cannula, wherein the fiber optic element is integrated into the sleeve in the form of a plurality of optical fibers 290, which form part of the fiber optic element of the system.

In the example shown in **Error! Reference source not found.**C, two optical fibers are integrated into the sleeve 280 surrounding the cannula. The distal ends of the optical fibers are aligned with the distal end of the cannula. The cannula will provide a suction force to the tissue with a constant pressure, thereby holding the tissue against the optical fiber ends with a constant force, thereby creating a strong optical connection between the tissue and optical fibers, which is necessary for a robust readout of the diffuse reflectance spectrum of the tissue.

The sleeve 280 may be retrofitted onto any existing cannula used to provide a suction force to a tissue.

When using two optical fibers that are separated by a given distance, the measured absorbance of the fluorescent agent is amplified such that the signal-to-noise ratio of the light signal improves, thereby improving the accuracy of the determined concentration of the fluorescent agent. Further, by using two, or more, optical fibers, the system determine a concentration of the fluorescent agent at a greater distance in front of the distal end of the cannula, i.e. deeper into the tissue, thereby providing for an earlier detection of when the boundary of the tissue is approaching.

In an example, the light source is coupled to a first optical fiber of the plurality of optical fibers and the input for receiving the light signal is coupled to a second optical fiber of the plurality of optical fibers.

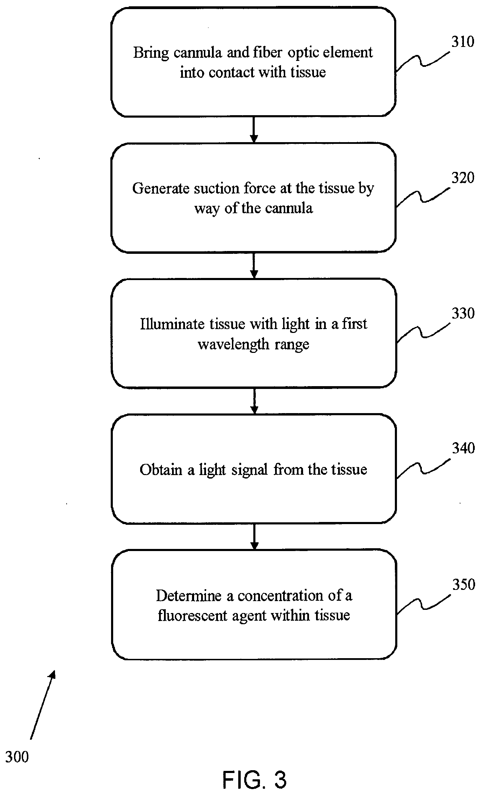

Figure 3 shows a method 300 for using the systems described above.

In step 310, the cannula and the fiber optic element are brought into contact with a tissue containing a fluorescent agent adapted to absorb light in a first wavelength range and emit light in a second, different wavelength range.

In step 320, the pressure within an internal cavity of the cannula is decreased, thereby providing a suction force to the tissue by way of the cannula, which brings and holds the tissue in contact with the fiber optic element.

In step 330, light in a first wavelength range is provided to the tissue, for example by way of a direct light source or by way of the fiber optical element coupled to the light source. Light in the first wavelength range will be absorbed by the fluorescent agent within the tissue of interest.

In step 340, a light signal is obtained from the tissue by way of the fiber optic element, the light signal comprising light in the first wavelength range and light in the second wavelength range.

In step 350, the concentration of the fluorescent agent within the tissue is determined based on the proportion of light in the first wavelength range in the light signal.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (100) for investigating a tissue (110) of a subject by way of diffuse reflectance spectroscopy, the tissue including a fluorescent agent adapted to absorb light in a first wavelength range and emit light in a second wavelength range, the second wavelength range being different to the first wavelength range, wherein the system comprises:
a cannula (120) having a distal end to be positioned adjacent the tissue, wherein the cannula comprises an internal cavity (130) configured to be connected to a pressure generator in order to generate a reduced pressure within the internal cavity, thereby providing a suction force to the tissue at the distal end of the cannula;
a light source (150) adapted to generate light (160) at the first wavelength range to be provided to the tissue;
a fiber optic element (170) to be positioned adjacent the tissue and adapted to receive a light signal from the fluorescent agent, the light signal comprising light at the first wavelength range and light at the second wavelength range; and
a processing system (180), the processing system comprising an input for receiving the light signal, and wherein the processing system is adapted to determine a concentration of the fluorescent agent in the tissue based on a proportion of light at the first wavelength range in the light signal.

2. The system (100) as claimed in claim 1, wherein determining the concentration of the fluorescent agent comprises measuring an absorbance of the fluorescent agent based on a comparison between the proportion of light generated by the light source in the first wavelength range and the proportion of light detected in the first wavelength range in the light signal.

3. The system (100) as claimed in any of claims 1 to 2, wherein the fiber optic element (170) is positioned relative to the cannula (120) such that a distal end of the fiber optic element aligns with a distal end of the cannula.

4. The system (100) as claimed in claim 3, wherein the fiber optic element (220) is provided within the internal cavity (210) of the cannula (200).

5. The system (100) as claimed in claim 3, wherein the system further comprises a sleeve (280) adapted to receive the cannula (260), wherein the fiber optic element (290) is integrated into the sleeve.

6. The system (100) as claimed in any of claims 1 to 5, wherein the fiber optic element (170) comprises a plurality of optical fibers.

7. The system (100) as claimed in claim 6, wherein the light source (150) is coupled to a first optical fiber of the plurality of optical fibers, and wherein the input for receiving the light signal is coupled to a second optical fiber of the plurality of optical fibers.

8. The system (100) as claimed in any of claims 1 to 7, wherein the light source (150) comprises:
one or more LEDs, and wherein the input for receiving the light signal comprises a photodiode, and/or
a white light source, and wherein the system further comprises an optical filter adapted to filter out light outside of the first wavelength range.

9. The system (100) as claimed in any of claims 1 to 8, wherein the processing system (180) is further adapted to identify a boundary between the tissue (110) and an adjacent tissue (190) based on the determined concentration of the fluorescent agent in the tissue.

10. The system (100) as claimed in claim 9, wherein the processing system (180) is adapted to receive an initial reading of the adjacent tissue by way of the fiber optic element, and wherein determining the concentration of the fluorescent agent is further based on the initial reading.

11. The system (100) as claimed in any of claims 9 to 10, wherein the processing system (180) is further adapted to generate an alert to be provided to a user based on the identified boundary.

12. The system (100) as claimed in claim 11, wherein the system further comprises a display for displaying the alert to the user.

13. The system (100) as claimed in any of claims 1 to 11, wherein the system further comprises a display for displaying the determined concentration of the fluorescent agent to a user.

14. The system (100) as claimed in any of claims 1 to 13, wherein the system is adapted for use when the tissue is being resected.

15. An arrangement comprising a fiber optic element (170) adapted to be connected to a system (100) for investigating a tissue (110) of a subject by way of diffuse reflectance spectroscopy, the tissue including a fluorescent agent adapted to absorb light in a first wavelength range and emit light in a second wavelength range, the second wavelength range being different to the first wavelength range, the fiber optic element comprising a connector for removably connecting the optical fiber element to a cannula such that it is positioned adjacent the tissue upon use of a cannula (120) having a distal end to be positioned adjacent tissue of a subject, wherein the cannula comprises an internal cavity (130) configured to be connected to a pressure generator in order to generate a reduced pressure within the internal cavity, thereby providing a suction force to the tissue at the distal end of the cannula and, optionally, wherein the connector comprises a sleeve for receiving the cannula and having attached thereto or integrated therein the optical fiber element.
